# EUROPÄISCHE PATENTANMELDUNG

(11) **EP 1 923 099 A1**
(43) Veröffentlichungstag der Anmeldung: **21.05.2008**
(21) Anmeldenummer: 07020385.6
(22) Anmeldetag: 18.10.2007
(51) Int. Cl.: A61N 1/375

(54) **Filterdurchführung für Implantate**

(30) Priorität: 17.11.2006 DE 102006054249
(71) Anmelder: BIOTRONIK CRM Patent AG, 6341 Baar (CH)
(72) Erfinder: Starke, Marcel, 12167 Berlin (DE)
(74) Vertreter: Lindner-Vogt, Karin L.

(57) **Zusammenfassung**

Die Erfindung liegt auf dem Gebiet medizinischer Implantate, wie z. B. Herzschrittmacher und Defibrillatoren. Zur Verbindung zwischen geräteinterner Elektronik und externen Komponenten wird eine neue hermetisch dichte Kontakt-Durchführung vorgeschlagen, wobei eine flache Keramikscheibe (1) als isolierender Grundträger dient, in der Öffnungen (3) angeordnet sind, in die verschiedene Elektrodenausführungen (4, 5, 6, 7) als Durchkontaktierungen eingelassen werden können. Mittels Metallflansch oder metallisierter Bedampfungszone (2) lässt sich die Keramikscheibe direkt auf das Implantatgehäuse (11) auflöten. Aktive und passive Zusatzkomponenten (8) können zusätzlich direkt auf die Keramik aufgebracht werden. In spezieller Ausführung der Erfindung ist der Grundträger als Mehrschicht-Keramik (9) ausgeführt, so dass Umverdrahtungsebenen und Abschirmungskomponenten (10) in die Durchführung integriert werden können. Die erfindungsgemäße Durchführung ermöglicht durch den Einsatz von standardisierten Keramik-Halbzeugen neue Aufbauvarianten, vor allen Dingen mit Ausrichtung auf multipolare Systeme.

## Beschreibung

### Filterdurchführung für Implantate

Die vorliegende Erfindung liegt auf dem Gebiet medizinischer Implantate mit elektronischen Komponenten, wie beispielsweise Herzschrittmacher, Defibrillatoren, Cardiovertern, Nervenstimulatoren oder Gehörgangsimplantate. Insbesondere betrifft die Erfindung die bei derartigen Geräten erforderlichen hermetisch dichten Durchführungen für die verschiedenen elektrischen Verbindungen zwischen Geräteinterner Elektronik und externen Komponenten. In spezieller Ausführungsform betrifft die Erfindung die für solche Durchführungen erforderliche Abschirmung bzw. Filterung elektromagnetischer Störungen (electromagnetic interference, EMI).

Weiterhin betrifft die vorliegende Erfindung ein Fertigungsverfahren zur Herstellung der erfindungsgemäßen Komponenten.

Implantierbare Geräte bestehen typischerweise aus einem Metallgehäuse mit einer darin eingelassenen Steckverbindung zur Aufnahme externer Anschlüsse z.B. für elektrische Stimulierungen oder Messung physiologischer Signale. Von besonderer Bedeutung ist dabei die hermetische Abdichtung der im Innenbereich angeordneten Komponenten (Steuerelektronik, Batterie) gegenüber den Umgebungsbedingungen, wie sie im Körperinnern des Trägers herrschen. Dies stellt besondere Anforderungen an die Durchführungen der elektrischen Verbindungen. Hier dürfen keine Korrosionseffekte auftreten, jede einzelne Durchkontaktierung muss absolut dicht gegenüber Gas- und Flüssigkeitsdiffusion sein.

Stand der Technik ist es, dass bei Durchführungen für Implantate Isolatoren aus Keramik als Träger eingesetzt werden. Diese werden überwiegend zylindrisch ausgeformt und als Formteile gepresst und gesintert. In der Regel wird über vorgefertigte Öffnungen in der Keramik mittels metallischer Kontaktstifte (Pins) die elektrische Durchkontaktierung hergestellt. Für den keramischen Isolator seinerseits ist über einen metallischen Flansch die Anschlussmöglichkeit an das Implantatgehäuse geschaffen. Pin, Isolationskeramik und Flansch sind dabei so angeordnet, dass die Pins von der Keramik umschlossen werden und die Keramik wiederum vom Flansch. Eine solche Durchführung ist beispielsweise beschrieben in den Dokumenten US 5,759,197 und US 5,836,992. Figur 1 zeigt eine solche Anordnung nach dem Stand der Technik.

Keramik, Flansch und Pins werden in der Regel durch einen Hartlötprozess mit Goldlot hermetisch dicht verlötet. Dafür muss die Isolationskeramik an den Lotflächen vorher partiell metallisiert werden. Diese Beschichtungstechnik ist aufwändig, weil dafür Einzelmasken eingesetzt werden müssen. Die beschriebene Form und die Spezialisierung der Einzelschritte verhindern daher für Durchführungen nach dem Stand der Technik kostengünstige Fertigungsverfahren und die Nutzung von kostengünstigen Halbzeugen.

Abweichend von der beschriebenen Bauform sind alternative Ansätze aus den Dokumenten US 5,620,476 und US 5,683,435 bekannt. Dort werden statt einzelner Durchführungen mit je einem Pin schmale Keramiken im Mehrlagenaufbau genutzt. Eine Anzahl verschiedener elektrischer Durchführungskontakte wird hier als Leiterbahnen auf inneren Schichten der Mehrlagenkeramik aufgebracht. Die streifenförmige Mehrlagenkeramik ihrerseits wird dann ringförmig von einem ovalen Flansch umschlossen, ähnlich wie der Kontaktstift im beschriebenen konventionellen Aufbau, und damit als vertikale Sammel-Durchführung am Gehäuse des Implantats eingelötet. Auch bei dieser Technik sind zahlreiche Einzelprozesse und Beschichtungen erforderlich. Insbesondere stellt die Abdichtung der vertikal eingesetzten Mehrschichtkeramik besondere Anforderungen an die Passgenauigkeit des ringförmig umschließenden Flanschs und die umlaufende Metallisierung der Keramik für den Einlötprozess.

Ein weiteres Problem bei Durchführungen für implantierbare Geräte ist die elektrische Störanfälligkeit gegenüber Einstreuungen durch externe elektromagnetische Felder. Wegen der einerseits steigenden Verstärkerempfindlichkeit und den andererseits zunehmenden externen Störeinflüssen (Funk bis GHz-Bereich, in der Nähe betriebene Handys mit starker Sendeleistung), wachsen die Anforderungen an eine Abschirmung durch EMI-Filterung. Für eine gute Filterung müssen passive oder aktive Bauelemente nahe an den direkten Eingängen positioniert werden und leitend mit den Pins und dem Flansch bzw. einem anderen Potential elektrisch kontaktiert werden.

Die Erfindung geht aus von dem beschriebenen Stand der Technik. Ihr liegt die Aufgabe zugrunde, eine alternative Durchführung bereitzustellen, die kostengünstig hergestellt werden kann und die genannten Anforderungen bezüglich einer hermetisch dichten Einfügung an das Implantatsgehäuse und Abschirmungsmöglichkeiten erfüllt. Als weitere Aufgabe liegt zugrunde, ein Fertigungsverfahren zur Herstellung einer erfindungsgemäßen Durchführung bereitzustellen. Diese Aufgaben werden bei einer Durchführung gemäß Oberbegriff des Anspruchs 1 erfüllt durch die charakterisierenden Merkmale des Anspruchs 1 und bei dem Fertigungsverfahren durch den Anspruch 12. Weitere Vorzüge und spezielle Ausgestaltungen der erfindungsgemäßen Durchführung und des Fertigungsverfahrens sind Gegenstand der Unteransprüche. Die Erfindung wird im Folgenden anhand bevorzugter Ausführungsbeispiele unter Bezug auf die Zeichnungen und die darin verwendeten Bezugszeichen näher erläutert.

Es zeigen:
- Fig. 1: Durchführung für Implantate nach dem Stand der Technik
- Fig. 2: Ausführungsform der erfindungsgemäßen Durchführung (Schnitt)
- Fig. 2a: Durchführung nach Fig. 2 mit Lot-Perle (solder-ball)
- Fig. 2b: Durchführung nach Fig. 2 mit Nagelkontakt (nail-head)
- Fig. 2c: Durchführung nach Fig. 2 mit Stift (pin)
- Fig. 3: Durchführung mit Kondensator zur EMI-Filterung
- Fig. 4: Durchführung nach Fig. 3 (Aufsicht) mit drei Kontakten
- Fig. 5: Ausführungsform als Mehrschichtkeramik mit Metallisierungslagen

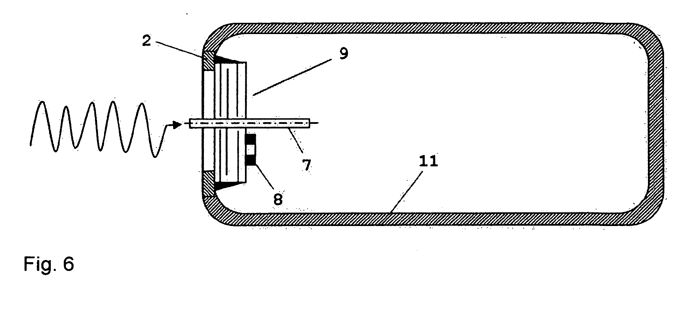

- Fig. 6: Abschirmungseffekt nach Einsatz ans Implantatsgehäuse

Um Durchführungen in Standardfertigungsverfahren kostengünstig herstellen zu können, muss der Gesamtaufbau an Standard-Fertigungsprozesse angepasst werden. Die Erfindung schlägt daher die Verwendung flacher Keramiken vor, die mit bekannten Verfahren hergestellt werden können. Dabei eignen sich bevorzugt Grünkeramiken wie ungebrannte LTCC- (Low Temperature Cofired Ceramics) oder ungebrannte HTCC-Keramiken (High Temperature Cofired Ceramics) als Ausgangsmaterialien. Weiterhin bevorzugt sind Al(2)O(3)-Keramiken verwendbar.

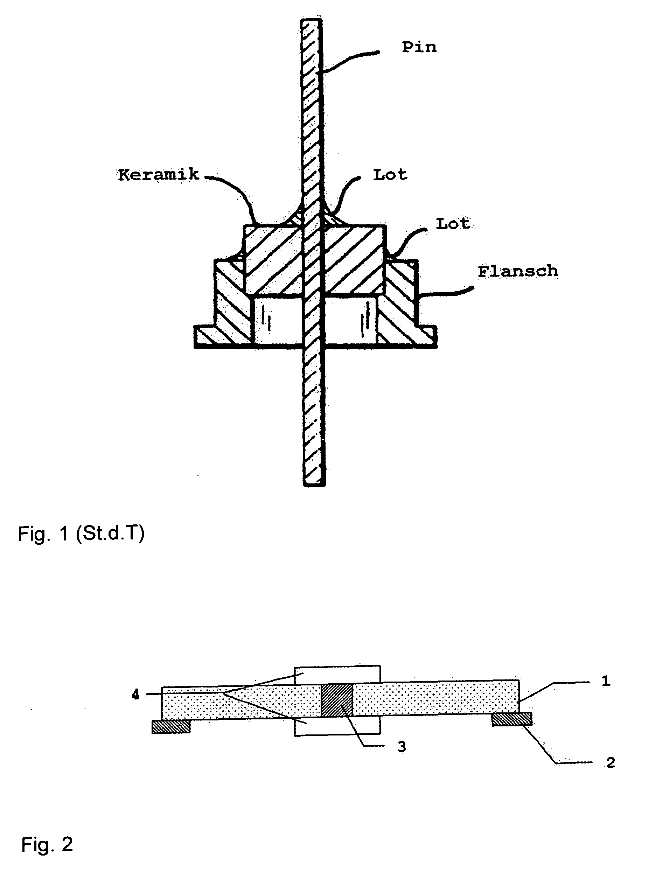

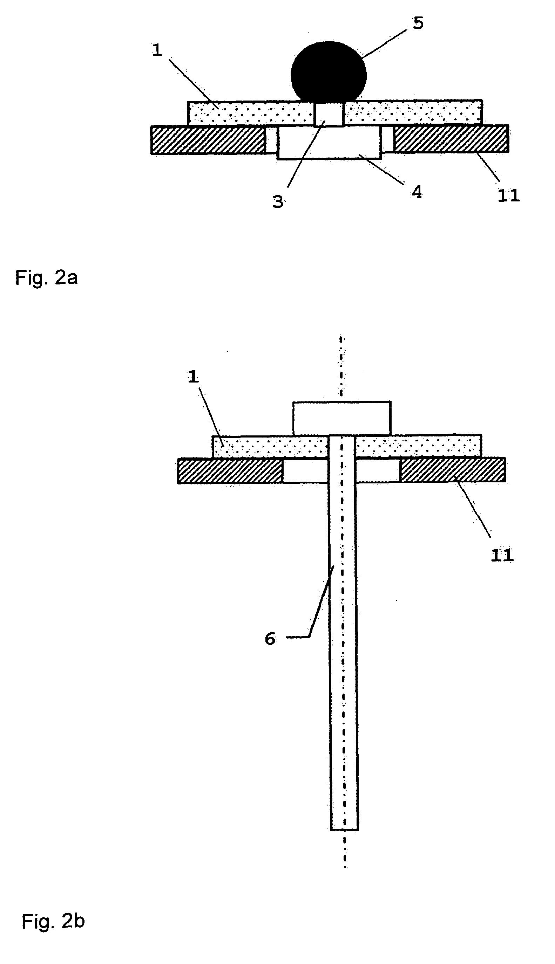

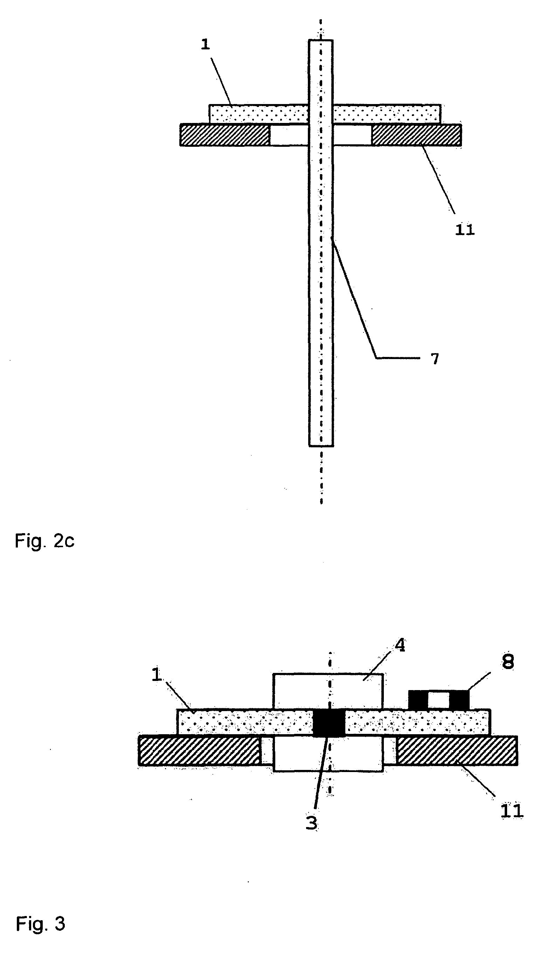

Fig. 2 zeigt als schematischen Schnitt den Aufbau einer ersten Ausführungsform der erfindungsgemäßen Durchführung. Als Isolator dient hier eine flache Keramik (1), die beispielsweise über einen Flansch (2) mit dem Implantat-Gehäuse z. B. mittels Lötprozess verbunden werden kann. Durch die besondere Bauform der Keramik ist es möglich, auf den Flansch zu verzichten und mittels Metallbeschichtung des Randbereichs die Keramik direkt an das Implantatgehäuse anzulöten.

Für die Durchkontaktierung weist die Keramik eine Öffnung (3) auf. Derartige Öffnungen sind beispielsweise durch eine Via-Technologie herzustellen, d. h. die Keramik wird in Standardtechnologien gebohrt oder vor einem Brennprozess gestanzt und die Bohrung wird durch Metallpasten aufgefüllt und damit eine elektrische Durchkontaktierung geschaffen.

Beidseitig der Öffnung sind im Beispiel nach Fig. 2 auf der Keramik Kontaktflächen (4) angeordnet. In Fig. 2a ist als alternative Ausführungsform einseitig statt einer der Kontaktfläche eine Lötperle (5) aufgebracht. Fig. 2b zeigt die erfindungsgemäße Durchführung mit nagelförmigem Kontakt (6), Fig. 2c mit einem üblichen Stiftkontakt (7).

In den Ausführungsbeispielen nach Fig. 2a, 2b und 2c ist der Flansch durch eine metallische Beschichtung ersetzt und die Keramik (1) darüber mit dem Implantatgehäuse (11) verbunden.

Der Vorteil der erfindungsgemäßen Durchführung besteht darin, dass es - im Gegensatz zu den kompliziert aufgebauten bisher gebräuchlichen Durchführungen - mit einem einfachen Verfahren hergestellt werden kann, welches durchaus bekannte Verfahrensabläufe zugrunde liegen hat. Das Fertigungsverfahren zur Herstellung der erfindungsgemäßen Kontakt-Durchführung weist folgende Abfolge von Schritten auf:
- Bereitstellen einer Flachkeramik
- Strukturieren der Flachkeramik, um eine Vielzahl flacher Keramikscheiben (1) zu schaffen, welche geeignet sind, als Träger zu dienen
- Beschichten der flachen Keramik, um Lötflächen zu schaffen
- Schaffen einer Lötverbindung an den Lötflächen
- Abtrennen der Keramikscheiben.

Der Vorteil dieses Fertigungsverfahrens liegt darin, dass eine Vielzahl von flachen Keramikscheiben (1) - die den Grundkörper der fertigen erfindungsgemäßen Kontakt-Durchführung bildet - aus einer vorgefertigten Flachkeramik im Nutzen, im Besonderen in einer Mehrfach-Nutzenstruktur, hergestellt werden. Je nach Größe der vorgefertigten Flachkeramik und der gewünschten Anzahl von Keramikscheiben (1) können mehr als 20, bevorzugt 20 bis 100, besonders bevorzugt 20 bis 50, erfindungsgemäße Durchführungen in einem Nutzen hergestellt werden. Damit kann wesentlich einfacher, schneller und mit einem hohen Automatisierungsgrad eine große Anzahl Kontakt-Durchführungen hergestellt werden. Ein weiterer Vorteil ist, dass noch vor dem letzten Verfahrensschritt (Abtrennen bzw. Vereinzelung der Keramikscheiben) im Nutzen ein Test beispielsweise auf Hermetizität (mittels beispielsweise HE-Lecktester) und/oder elektromagnetischer Verträglichkeit durchgeführt werden kann.

Vorzugsweise beinhaltet der 2. Arbeitschritt des Fertigungsverfahrensablaufs "Strukturieren der Flachkeramik" Arbeitschritt "Herstellen mindestens einer Öffnung". Die Öffnung (3) kann durch spangebendes Bohren oder durch spanfreies Lasern oder Stanzen erfolgen. Es kommen für diesen Arbeitschritt auch andere geeignete spangebende oder spanfreie Verfahren in Frage. Die Öffnung (3) dient dazu, einen elektrischen Kontakt (4, 5, 6, 7) vom Inneren eines Gehäuses nach außerhalb des Gehäuses zu ermöglichen.

Erfindungsgemäß wird die Flachkeramik beispielsweise mittels Stanzen, Lasern, Ritzen oder einem anderen dem Fachmann bekannten Verfahren strukturiert.

Die Beschichtung dient der Schaffung von Lötflächen, um elektrisch aktive und/oder passive Komponenten (8) wie Kondensatoren, Spulen oder Mikrochips elektrisch zu verbinden. Diese aktiven und/oder passiven Bauteile werden zur Abschirmung des Gehäuseinneren gegen externe elektromagnetische Strahlungen und/oder zur Übertragung von HF-Signalen für die Telemetrie über größere Distanzen eingesetzt. Zur Übertragung der HF-Signale werden Chips mit integrierten Sende- /Empfangsteilen eingesetzt. Es hat sich als vorteilhaft herausgestellt, diese Telemetrie-Bauteile direkt an einer Kontaktdurchführung zu platzieren, da die Übertragung von HV-Signalen in Implantatsgehäuse hinein und aus diesem heraus unter Umgehung des Implantatsgehäuses wesentlich erleichtert wird. Dadurch kann das Übertragen von HF-Signalen mit einer erheblich geringeren Sendeenergie erfolgen, was die Lebensdauer des Implantates erhöht.

Die Bestückung mit diesen Bauteilen erfolgt nach der Herstellung einer Lötverbindung und umfasst den Fertigungsschritt "Weichlöten der Bauteile", um einen elektrische Kontakte herzustellen. Die Lötflächen können im Übrigen auch zur elektrischen Verbindung zwischen einem schweißbaren Flansch und einem Kontakt (4, 5, 6, 7) dienen. Deshalb sind die Lotflächen für Hart- oder Weichlot geeignet, beispielsweise auf Basis von Niob oder Titan, da der Flansch und die Durchführung hermetisch dicht verlötet werden müssen, was bei einem Weichlotverfahren nicht gewährleistet ist. Der Einsatz von elektrisch leitfähigen Klebern ist auch denkbar.

Die Beschichtungen für die Lötflächen werden mit Hilfe von Schablonen einfach und für automatische Verfahren geeignet hergestellt.

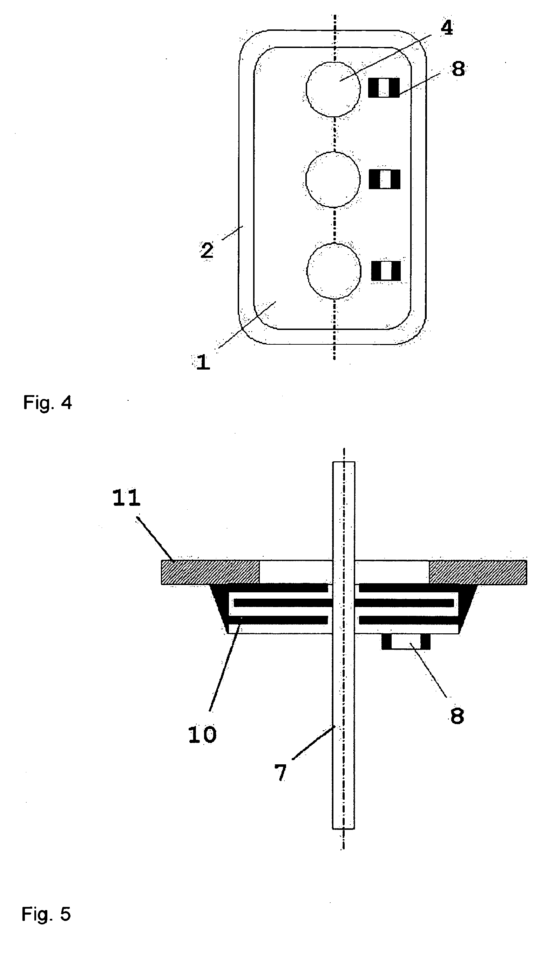

Fig. 3 zeigt eine erweiterte Ausführungsform der keramischen Durchführung, bei der weitere Komponenten von der Keramik getragen werden. In diesem Beispiel ist auf einer Bestückungsseite als SMT-Bauteil (surface-mount-technology) ein Kondensator (8) aufgebracht, der beispielsweise zur EMI-Filterung eingesetzt ist. Fig. 4 zeigt eine entsprechende Aufsicht auf eine solche Ausführung mit drei nebeneinander angeordneten Kontaktflächen (4) und zugeordneten Kondensatoren (8). Die elektrischen Verbindungen der Kondensatoren, z. B. zum Anschluss-Flansch, können z. B. durch auf die Keramikoberfläche aufgedampfte Leiterbahnen erfolgen (nicht dargestellt). Für solche Bestückungen mit aktiven und passiven Elementen können Standardverfahren der Elektronik verwendet werden (SMT-Bestückung im Nutzen mittels "Pick&Place"-Geräten).

Als Isolator können sowohl kompakte, einschichtige Keramikscheiben, als auch Mehrschichtkeramiken eingesetzt werden. Bei Mehrschichtkeramiken können in den Zwischenlagen der Keramik Verdrahtungsebenen eingebracht werden, die auch zur Kontaktierung von aktiven und passiven Komponenten (8) eingesetzt werden können. Fig. 5 zeigt eine solche Ausführungsform der Erfindung mit Mehrschichtkeramik (9). Ein weiterer Vorteil dieser Ausführungsform ist die zusätzliche Abschirmungswirkung. Hierzu können zusätzliche Metallisierungsebenen (10) eingebracht werden, die beispielsweise in elektrischer Verbindung zum Potential des leitfähigen Implantatsgehäuses (11) stehen, so dass ein Faraday-Käfig gebildet wird. Werden die metallisierten Keramik-Lagen entsprechend dünn zueinander ausgelegt, entsteht eine flache Kapazität, die als Eingangsfilter arbeitet. Dabei kann die Filterwirkung auf spezielle Frequenzbereiche (Wellenlänge λ der einfallenden Störungssignale) dadurch optimiert werden, dass ein geeigneter Abstand der Lagen vorgegeben ist (zum Beispiel: Lagenabstand D als ganzzahliger Bruchteil der Wellenlänge: D = λ/2,...,λ/100).

Die erfindungsgemäße Durchführung ermöglicht durch den Einsatz von Keramik-Halbzeugen neue Aufbauvarianten, vor allen Dingen mit Ausrichtung auf multipolare Systeme. Dabei bietet die Durchführung eine hohe Design-Flexibilität und Unabhängigkeit von speziellen Werkzeugen. Zudem lassen sich durch den erfindungsgemäßen Aufbau der Durchführung Schaltungselemente einfach und kostengünstig direkt auf der Isolatorkeramik in Standardtechnologie nutzen. Bei Einsatz von Mehrlagen können Abschirmungskomponenten und Umverdrahtungsebenen in die Durchführung integriert werden, wobei der Aufbau eine kostengünstige automatisierbare Anwendung von Flachtechnologien (Pastendruck, einfache Maskentechniken) ermöglicht.

## Patentansprüche

1. Hermetische Kontakt-Durchführung für implantierbare elektronische Geräte, wie z. B. Herzschrittmacher, Defibrillatoren o. ä., wobei ein isolierender Träger (1) jeweils mindestens eine Öffnung (3) zur Durchführung eines Kontakts (4, 5, 6, 7) aufweist, sowie einen löt- oder schweißbaren Bereich (2), über den der Träger (1) mit dem Gehäuse (11) des implantierbaren Geräts elektrisch leitend verbunden ist,
**dadurch gekennzeichnet, dass**
der Träger (1) als flache Keramikscheibe ausgeführt ist, so dass das Gehäuse (11) des implantierbaren Geräts den Träger (1) nicht umschließt.

2. Kontaktdurchführung nach Anspruch 1, **dadurch gekennzeichnet, dass** auf deren Oberfläche Areale zum Beispiel mittels Metall-Bedampfung zur Aufnahme aktiver und/oder passiver elektrischer Komponenten (8), zum Beispiel in SMT-Ausführung, ausgestaltet sind.

3. Kontaktdurchführung nach Anspruch 2, **dadurch gekennzeichnet, dass** die aktiven und/oder passiven elektrischen Komponenten (8) zur Abschirmung des Gehäuseinneren gegen externe elektromagnetische Strahlungen und/oder zur Übertragung von HF-Signalen für die Telemetrie über größere Distanzen eingesetzt werden.

4. Kontaktdurchführung nach einem der Ansprüche 1 bis 3, **dadurch gekennzeichnet, dass** der Träger (1) als Mehrschichtkeramik (9) ausgeführt ist.

5. Kontakt-Durchführung nach Anspruch 4, **dadurch gekennzeichnet, dass** die Mehrschichtkeramik (9) zumindest eine interne Lage aufweist, die als Verdrahtungsebene zur Verbindung aktiver und/oder passiver elektrischer Komponenten (8) ausgestaltet ist.

6. Kontakt-Durchführung nach Anspruch 4 oder 5, **dadurch gekennzeichnet, dass** die Mehrschichtkeramik (9) eine oder mehrere interne Lagen (10) aus leitfähigem Material aufweist, die zur Abschirmung gegen elektromagnetische Störungen ausgestaltet sind.

7. Kontakt-Durchführung nach Anspruch 6, **dadurch gekennzeichnet, dass** die eine oder mehrere interne Lagen (10) zur Abschirmung beispielsweise über den leitfähigen Bereich (2) zur Bildung eines Faraday-Käfigs mit dem Potential des Implantatgehäuses (11) elektrisch verbunden sind.

8. Kontakt-Durchführung nach Anspruch 6, **dadurch gekennzeichnet, dass** interne Lagen (10) zur Abschirmung als Kondensatorflächen ausgebildet sind.

9. Kontakt-Durchführung nach Anspruch 7 oder 8, **dadurch gekennzeichnet, dass** interne Lagen (10) zur Abschirmung als Zwischenebenen der Mehrschichtkeramik (9) in vorgegebenem Abstand zueinander stehen.

10. Kontakt-Durchführung nach Anspruch 9, **dadurch gekennzeichnet, dass** der vorgegebene Abstand zur Dämpfung definierter Wellenlängen (λ) einfallender elektromagnetischer Störstrahlung als ganzzahliger Bruchteil der Wellenlänge (λ/n) ausgelegt ist.

11. Kontakt-Durchführung nach einem der Ansprüche 1 bis 10, **dadurch gekennzeichnet, dass** der löt- oder schweißbare Bereich (2) als metall-bedampfter Außenrand der flachen Trägerkeramik (1) ausgebildet ist.

12. Verfahren zum Herstellen einer Kontakt-Durchführung nach einem der Ansprüche 1 bis 11, enthaltend folgende Abfolge von Schritten:
- Bereitstellen einer Flachkeramik
- Strukturieren der Flachkeramik, um eine Vielzahl flacher Keramikscheiben zu schaffen, welche geeignet sind, als Träger (1) zu dienen
- Beschichten der flachen Keramikscheiben, um Lötflächen zu schaffen
- Schaffen einer Lötverbindung an den Lötflächen, und
- Abtrennen der Keramikscheiben

13. Verfahren nach Anspruch 12, **dadurch gekennzeichnet, dass** das Strukturieren der Flachkeramik zusätzlich folgenden Schritt umfasst:
- Herstellen mindestens einer Öffnung (3), welche geeignet ist, einen Kontakt (4, 5, 6, 7) durchzuführen.

14. Verfahren nach einem der Ansprüche 12 oder 13, **dadurch gekennzeichnet, dass** das Strukturieren der Flachkeramik beispielsweise durch Stanzen, Lasern oder Ritzen erfolgt.

15. Verfahren nach Anspruch 12, **dadurch gekennzeichnet, dass** die Beschichtung mit Hilfe von Masken oder Schablonen erfolgt.

16. Verfahren nach Anspruch 12, **dadurch gekennzeichnet, dass** nach dem Schaffen einer Lötverbindung eine Bestückung der Kontakte (4, 5, 6, 7) mit aktiven und/oder passiven elektrischen Komponenten (8) erfolgt.
